(19) Europäisches Patentamt
European Patent Office
Office européen des brevets

(11) **EP 3 714 789 A1**

(12) **EUROPEAN PATENT APPLICATION**

(43) Date of publication:
**30.09.2020  Bulletin 2020/40**

(51) Int Cl.:
*A61B 5/145* (2006.01)        *A61B 5/1455* (2006.01)
*A61B 5/053* (2006.01)        *A61B 5/00* (2006.01)

(21) Application number: **20165194.0**

(22) Date of filing: **24.03.2020**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **25.03.2019   KR 20190033932**

(71) Applicant: **Samsung Electronics Co., Ltd.
Gyeonggi-do 16677 (KR)**

(72) Inventors:
• **LEE, So Young
  Daejeon (KR)**
• **CHOI, Ka Ram
  Gyeonggi-do (KR)**

(74) Representative: **Grünecker Patent- und
Rechtsanwälte
PartG mbB
Leopoldstraße 4
80802 München (DE)**

(54) **HEALTHCARE APPARATUS AND HEALTHCARE METHOD**

(57)   Provided is a healthcare apparatus including a pulse wave sensor including a light source configured to emit light toward a user and a detector configured to detect light reflected or scattered from the user, the pulse wave sensor being configured to measure a pulse wave signal based on the detected light, and a processor configured to obtain an optical path length change of blood of the user based on the pulse wave signal, and to predict whether the user has a health problem based on the obtained optical path length change.

## FIG. 1

EP 3 714 789 A1

## Description

## BACKGROUND

### 1. Field

Example embodiments of the present application relate to technology for predicting whether a user has health problems based on a pulse wave signal, and more particularly to technology for predicting hyperosmolar coma.

### 2. Description of the Related Art

Diabetes is a chronic disease that causes various complications and is very difficult to cure, such that people with diabetes are advised to check their blood glucose regularly to prevent complications. In particular, when insulin is administered to control blood glucose, the blood glucose levels have to be closely monitored to avoid hypoglycemia and control insulin dosage. An invasive method of finger pricking is generally used to measure blood glucose levels. However, while the invasive method may provide high reliability in measurement, it may cause pain and inconvenience as well as an increased risk of disease infections due to the use of injection. Recently, research has been conducted on methods of non-invasively estimating bio-information, such as blood glucose, by performing spectrum analysis using a spectrometer without blood sampling. It is known that about 1% of diabetic patients are at risk of hyperosmolar coma, and if no proper action is taken immediately, they may have brain damage or experience fatal illness.

## SUMMARY

One or more example embodiments provide an apparatus and method for predicting whether a user has health problems based on a pulse wave signal, and more particularly for predicting hyperosmolar coma.

According to an aspect of an example embodiment, there is provided a healthcare apparatus including a pulse wave sensor including a light source configured to emit light toward a user and a detector configured to detect light reflected or scattered from the user, the pulse wave sensor being configured to measure a pulse wave signal based on the detected light, and a processor configured to obtain an optical path length change of blood of the user based on the pulse wave signal, and to predict whether the user has a health problem based on the obtained optical path length change.

The processor may be further configured to extract an alternating current component of the pulse wave signal, and obtain the optical path length change based on a maximum value of the extracted alternating current component and a minimum value of the extracted alternating current component.

The processor may be further configured to predict a risk of hyperosmolar coma of the user by monitoring a variation in red blood cell size of the user based on the obtained optical path length change.

The processor may be further configured to predict the risk of hyperosmolar coma of the user based on the obtained optical path length change exceeding a predetermined reference value.

The predetermined reference value my include reference interval information for each of a plurality of levels corresponding to a degree of risk of hyperosmolar coma, and the processor may be further configured to predict the degree of risk of hyperosmolar coma based on the obtained optical path length change based on the reference interval information for each of the plurality of levels.

The apparatus may further include an action interface, the action interface being configured to perform an action corresponding to the predicted health problem of the user based on the prediction that the user has the health problem.

The action interface may be further configured to output an alarm by using at least one of a display, a speaker, and a haptic device based on the prediction that the user has the health problem.

The action part may be further configured to output at least one of information on the predicted health problem and an action corresponding to a degree of risk of the predicted health problem based on the prediction that the user has the health problem.

The information on the predicted health problem may include at least one of the optical path length change, impedance information, information on whether a blood glucose concentration is abnormal, information on whether there is a risk of hyperosmolar coma, and a degree of risk of hyperosmolar coma, and the action may include at least one of medication intake, fluid intake, rest, exercise, and hospital information.

The action interface may be further configured to transmit information on the health problem to an electronic device by a communication interface based on the prediction that the user has the health problem.

According to another aspect of an example embodiment, there is provided a healthcare method including measuring a pulse wave signal by emitting light onto an a user and detecting light reflected or scattered from the user, obtaining an optical path length change of blood based on the pulse wave signal, and predicting whether the user has a health problem based on the obtained optical path length change.

The obtaining of the optical path length change may include extracting an alternating current component of the pulse wave signal, and obtaining the optical path length change based on a maximum value of the extracted alternating current and a minimum value of the extracted alternating current component.

The predicting whether the user has the health problem may include predicting hyperosmolar coma by monitoring a change in red blood cell size of the user based on the obtained optical path length change.

The predicting whether the user has the health problem may include predicting that the user is at risk of hyperosmolar coma based on the obtained optical path length change exceeding a predetermined reference value.

The predetermined reference value may include reference interval information for each of a plurality of levels corresponding to a degree of risk of hyperosmolar coma, and the predicting whether the user has a health problem may include predicting the degree of risk of hyperosmolar coma based on the obtained optical path length change based on the reference interval information for each of the plurality of levels.

The method may further include performing an action corresponding to the health problem of the user based on the prediction that the user has the health problem.

The performing of the action may include outputting an alarm by at least one of a display, a speaker, and a haptic device based on the prediction that the user has the health problem.

The performing of the action may include outputting at least one of information on the predicted health problem of the user and an action corresponding to a degree of risk of the health problem based on the prediction that the user has the health problem.

The information on the predicted health problem may include at least one of the optical path length change, impedance information, information on whether a blood glucose concentration is abnormal, information on whether there is a risk of hyperosmolar coma, and a degree of risk of hyperosmolar coma, and the action may include at least one of medication intake, fluid intake, rest, exercise, and hospital information.

The performing of the action may include transmitting information on the health problem to an electronic device by a communication interface based on the prediction that the user has the health problem.

According to yet another aspect of an example embodiment, there is provided a healthcare apparatus including a pulse wave sensor including a light source configured to emit light onto a user and a detector configured to detect light reflected or scattered from the user, the pulse wave sensor being configured to measure a pulse wave signal based on the detected light, an impedance sensor configured to measure an impedance of the user, and a processor configured to obtain an optical path length change of blood based on the pulse wave signal, obtain a body fluid variation based on the measured impedance, and predict whether the user has a health problem based on the optical path length change and the body fluid variation.

The processor may be further configured to predict a risk of hyperosmolar coma by monitoring a variation in red blood cell size of the user based on the optical path length change and the body fluid variation.

The processor may be further configured to combine the optical path length change and the body fluid variation, and the processor may be configured to predict that there is a risk of hyperosmolar coma based on the combination of the optical path length change and the body fluid variation exceeding a predetermined reference value.

The processor may be further configured to assign a weighted value to each of the optical path length change and the body fluid variation based on at least one characteristic of the user, and to combine the optical path length change and the body fluid variation, to each of which the weighted value is assigned.

The apparatus may further include an action interface configured to perform an action corresponding to the health problem of the user based on the prediction that the user has the health problem.

According to yet another aspect of an example embodiment, there is provided a healthcare including a pulse wave sensor including a light source configured to emit light toward a user and a detector configured to detect light reflected or scattered from the user, the pulse wave sensor being configured to measure a pulse wave signal based on the detected light, and a processor configured to obtain an optical path length change of blood of the user based on the pulse wave signal, and predict a risk of hyperosmolar coma of the user based on the obtained optical path length change exceeding a predetermined reference value.

The predetermined reference value may correspond to at least one characteristic of the user, the at least one characteristic including being diabetic and non-diabetic.

BRIEF DESCRIPTION OF THE DRAWINGS

The above and/or other aspects, features, and advantages of example embodiments will be more apparent from the following description taken in conjunction with the accompanying drawings, in which:

FIG. 1 is a block diagram illustrating a healthcare apparatus according to an example embodiment;
FIG. 2 is a diagram illustrating a configuration of a processor of the healthcare apparatus of FIG. 1;
FIGS. 3A and 3B are diagrams illustrating examples of a photoplethysmography (PPG) signal;
FIGS. 4A and 4B are diagrams illustrating examples of actions made in response to a prediction result of a health problem;
FIG. 5 is a block diagram illustrating a healthcare apparatus according to an example embodiment;
FIGS. 6A and 6B are diagrams illustrating a wearable device according to the example embodiment of FIG. 5;
FIG. 7 is a flowchart illustrating a healthcare method according to an example embodiment; and
FIG. 8 is a flowchart illustrating a healthcare method according to an example embodiment.

DETAILED DESCRIPTION

Example embodiments will be described with references to the following detailed description and drawings. Advantages and features of the present disclosure, and a method of achieving the same will be more clearly understood from the following example embodiments described in detail with reference to the accompanying drawings. Throughout the drawings and the detailed description, unless otherwise described, the same drawing reference numerals will be understood to refer to the same elements, features, and structures.

It will be understood that, although the terms "first," "second," etc. may be used herein to describe various elements, these elements should not be limited by these terms. These terms are only used to distinguish one element from another. In addition, unless explicitly described to the contrary, an expression such as "comprising" or "including" will be understood to imply the inclusion of stated elements but not the exclusion of any other elements. Expressions such as "at least one of," when preceding a list of elements, modify the entire list of elements and do not modify the individual elements of the list. For example, the expression, "at least one of a, b, and c," should be understood as including only a, only b, only c, both a and b, both a and c, both b and c, or all of a, b, and c. Also, the terms, such as "part" or "module," etc., should be understood as a unit that performs at least one function or operation and that may be embodied as hardware, software, or a combination thereof.

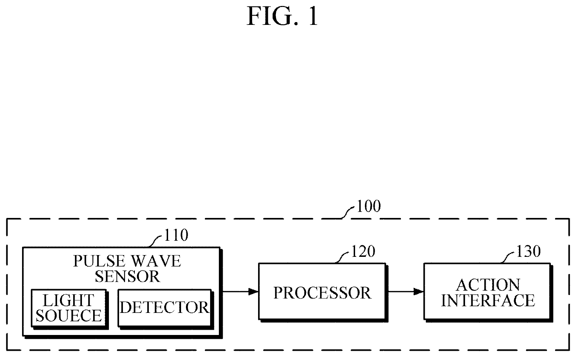

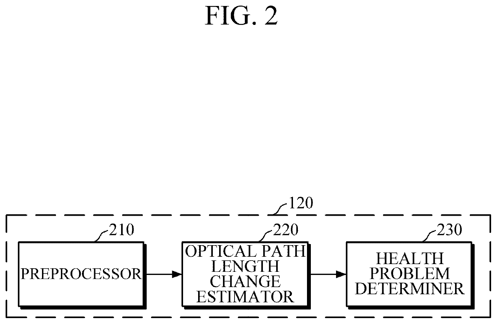

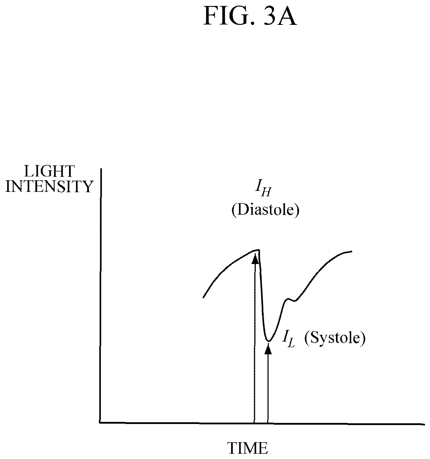

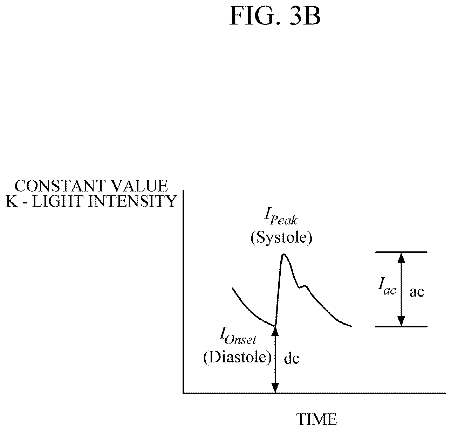

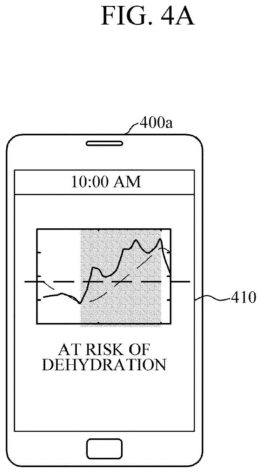

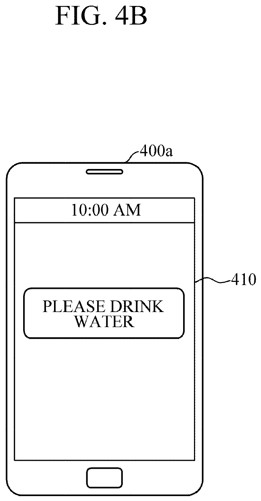

FIG. 1 is a block diagram illustrating a healthcare apparatus according to an example embodiment. FIG. 2 is a diagram illustrating a configuration of a processor of the healthcare apparatus of FIG. 1. FIGS. 3A and 3B are diagrams illustrating examples of a PPG signal. FIGS. 4A and 4B are diagrams illustrating examples of actions made in response to a prediction result of a health problem.

The healthcare apparatus 100 may be embedded in an electronic device such as a smartphone, a tablet personal computer (PC), a desktop computer, a laptop computer, and the like, or in a medical device used in a specialized medical institution. The healthcare apparatus 100 may be manufactured as an independent device, such as a wristwatch-type wearable device, a bracelet-type wearable device, a wristband-type wearable device, a ring-type wearable device, a glasses-type wearable device, a headband-type wearable device and the like, which may be worn on an object.

Referring to FIG. 1, the healthcare apparatus 100 includes a pulse wave sensor 110, a processor 120, and an action interface 130.

The pulse wave sensor 110 may emit light onto an examination portion of an object such as a user, and may measure a pulse wave signal, including a PPG signal, by detecting light reflected or scattered from the examination portion.

For example, the pulse wave sensor 110 may include a light source which emits light onto an object, and a detector which detects scattered or reflected light when light, emitted by the light source, is scattered or reflected from, for example, a body tissue of the object such as the surface of skin or blood vessels that is irradiated by the light emitted from the light source, and converts the detected light into an electric signal to output the signal.

The light source may include a light emitting diode (LED), a laser diode (LD), a fluorescent body, and the like, but is not limited thereto. The detector may include a photo diode, a photo transistor (PTr), an image sensor (e.g., complementary metal oxide semiconductor (CMOS) image sensor), and the like, but is not limited thereto. The pulse wave sensor 110 may include a plurality of arrays of light sources and/or detectors. In this case, the light sources may be formed to emit light of different wavelengths, and may be positioned at different distances from the detectors.

The processor 120 may be electrically connected to the pulse wave sensor 110. The processor 120 may receive the pulse wave signal from the pulse wave sensor 110, and may predict whether a user has a health problem based on the received pulse wave signal. In this case, the health problem may include risk of diabetes, an abnormal blood glucose level, risk of hyperosmolar coma, and the like, but is not limited thereto. The processor 120 may monitor a variation in red blood cell size by analyzing the pulse wave signal, and may determine whether a diabetic patient is at risk of hyperosmolar coma based on the monitoring result.

Referring to FIG. 2, the processor 120 according to an example embodiment includes a preprocessor 210, an optical path length change estimator 220, and a health problem determiner 230.

The preprocessor 210 may preprocess the pulse wave signal input by the pulse wave sensor 110. For example, upon receiving an electrical current signal from the pulse wave sensor 110, the preprocessor 110 may convert the current signal into a voltage signal and may amplify the voltage signal. The preprocessor 210 may also remove ambient noise by filtering, smoothing, and the like. For example, the preprocessor 210 may extract an alternating current (AC) component by filtering a direct current (DC), and may extract a direct current component by filtering an alternating current. In this case, the preprocessor 210 may obtain an original signal by removing motion noise from the pulse wave signal using a Kalman filter, and may also estimate a shape of the pulse wave signal by using the Kalman filter after passing the pulse wave signal through a low-pass filter (LPF), a high-pass filter (HPF), a band-pass filter (BPF), and the like.

The optical path length change estimator 220 may estimate an optical path length change by using an AC component and/or a DC component of the pulse wave signal. FIG. 3A illustrates an example of a pulse wave signal obtained as a result of preprocessing performed by the preprocessor 210. FIG. 3B is a diagram illustrating an example of a waveform of the pulse wave signal which is obtained by subtracting a light intensity of the pulse wave signal of FIG. 3A from an arbitrary constant k and inverting the pulse wave signal.

For example, the optical path length change estimator 220 may estimate an optical path length change using FIG. 3B and the following Equation 1.

[Equation 1]

$$\Delta d = abs \left[ 1 - \frac{I_{ac}}{k - I_{onset}} \right]$$

Herein, $\Delta d$ denotes the optical path length change, Iac denotes a difference between a maximum value $I_{peak}$ and a minimum value $I_{onset}$ of the AC component, and k denotes the arbitrary constant.

The health problem determiner 230 may determine whether there is a health problem based on the estimated optical path length change. In this case, a health problem to be monitored may include a variation in red blood cell size, an abnormal blood glucose concentration, risk of hyperosmolar coma, and the like, but is not limited thereto.

Generally, when the intake of sugar, dehydration, and the like leads to a sharp increase in blood glucose concentration, osmotic pressure may increase and the diameter of the red blood cells may be reduced. In this case, as the size of the red blood cells decreases, the optical path length of light, which is emitted onto an estimation portion of a user and passes through the blood vessels, may change. That is, the change in optical path length may have a correlation with the variation in red blood cell size, the blood glucose concentration, and the like. In this case, when hyperosmotic pressure is caused by a sharp increase in blood glucose concentration, diabetic patients may suffer from severe dehydration and metabolic disorders in the body, which may lead to coma or be fatal.

As described above, the health problem determiner 230 may predict a risk of hyperosmolar coma by applying a predetermined reference value based on a correlation between the change in optical path length and the variation in red blood cell size.

For example, for a user with diabetes or at risk of diabetes, if the estimated optical path length change exceeds a predetermined reference value, the health problem determiner 230 may predict that there is a risk of hyperosmolar coma. In another example, based on reference interval information for each plural levels which are classified according to a degree of risk of hyperosmolar coma, the health problem determiner 230 may predict the degree of risk of hyperosmolar coma according to an optical path length change estimated for each user.

In this case, the predetermined reference value and the reference interval information for each level may be defined in consideration of a user's characteristics. For example, for a diabetic patient, the reference value may be set to a relatively low value, and for a non-diabetic user, the reference value may be set to a relatively high value. Accordingly, for the same change in optical path length, the health problem determiner 230 may predict a risk of hyperosmolar coma for a user with diabetes, but for non-diabetic users, the health problem determiner 230 may determine that their health conditions are normal.

The action interface 130 may perform a necessary action in response to a prediction made by the processor 120 that a user has a health problem. In this case, at least some of the functions of the action interface 130 may also be included in the processor 120.

For example, by using a display, a speaker, a haptic device, and the like, the action interface 130 may provide a prediction result, and/or an alarm, an action, and the like if it is determined that there is a health problem based on the prediction result.

For example, the action interface 130 may visually output the prediction result including the estimated optical path length change, the variation in red blood cell size, the blood glucose concentration, information on whether there is a risk of hyperosmolar coma, a degree of risk of hyperosmolar coma, and the like. Further, simultaneously or separately from the visual output of the prediction result, the action interface 130 may output an audio prediction result, for example by a voice of a person using a speaker.

In another example, in response to determination that there is a health problem, the action part 130 may visually/non-visually output a warning message to raise a user's awareness. For example, while visually displaying the warning message on a display, the action interface 130 may output the warning message by audio such as a voice of a person. The action interface 130 may provide the user with a regular sound signal, an alarm signal such as vibrations and tactile sensation, and the like. In this case, in order to allow the user to easily recognize the warning message, the action interface 130 may output the warning message by differently combining colors and fonts of letters of the warning message, the volume of the sound signal, the amplitude of a beep signal, an interval for generating the beep signal, the strength of vibration and tactile sensation, and the like according to a degree of health risk.

For example, FIG. 4A illustrates a smartphone 400a including the healthcare apparatus 100. As illustrated in FIG. 4A, the action interface 130 may output a warning message, such as "at a risk of dehydration," to notify that there is a risk of dehydration due to hyperosmolar coma on a display 410 of the smartphone 400a at a current time 10:00 AM

Further, as illustrated in FIG. 4A, the action interface 130 may visually output a waveform of the pulse wave signal, which is measured before and after 10:00 AM, on the upper portion of the display 410. In this case, the action interface 130 may output, if necessary, other detailed information including the optical path length change, the variation in red blood cell size, an osmotic pressure level, and the like. In this case, the display 410 may be formed as a touch panel for touch input, which allows a user to view detailed information, actions, and the like by a touch input.

In another example, in response to determination that there is a health problem, the action interface 130 may provide actions which may be made urgently by a user. In this case, the actions may include various types of information, such as fluid intake, intake of medication such as electrolyte, insulin, and the like, rest, exercise, hospital information, and the like, which may urgently need to be taken according to the predicted degree of health risk.

Referring to FIG. 4B, in the case where a user is at risk of hyperosmolar coma, the action interface 130 may output a message, such as "please drink water," on the display 410 of the smartphone 400a. In this case, the action interface 130 may enable the user to more easily recognize the message by causing the message to blink at predetermined time intervals, or by changing the color of the message. In this case, as illustrated in FIG. 4A, when a predetermined time elapses after the warning message is provided, the action interface 130 may automatically provide actions. The action interface 130 may also provide the actions in response to a user's request for actions by a touch input and the like, or without providing the warning message.

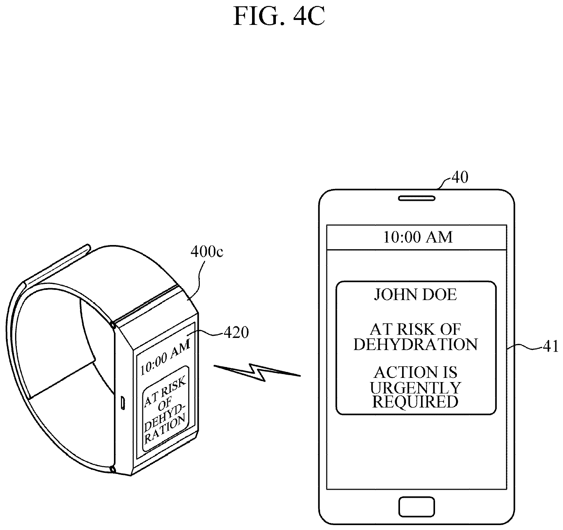

Referring to FIG. 4C, the action interface 130 may transmit information on a user's health problem to an electronic device 40 of a person concerned by using a communication interface, so that the person may rapidly make necessary actions. As illustrated in FIG. 4C, if a wearable device 400c including the healthcare apparatus 100 predicts that a user is at risk of hyperosmolar coma, the action interface 130 may transmit information on the health problem to the electronic device 40 of the person concerned while outputting an alarm message on a display 420. However, the action interface 130 is not limited thereto, and may transmit the health problem information to the electronic device 40 of the person concerned immediately without outputting an alarm message separately. In order to enable the person concerned to rapidly make a response action upon receiving the health problem information, the electronic device 40 of the person concerned may output the health problem information by using various output means, for example, the display 41, a speaker, a vibration generator, and the like. In this case, the person concerned may include a medical institution, a family doctor of a medical institution, a guardian, and the like. Further, the electronic device 40 may include various devices such as a smartphone, a medical institution server, a desktop computer, a laptop computer, a haptic device, a beep signal generator, and the like.

In this case, the communication interface may access a communication network by using communication techniques such as Bluetooth communication, Bluetooth Low Energy (BLE) communication, near field communication (NFC), WLAN communication, Zigbee communication, infrared data association (IrDA) communication, Wi-Fi Direct (WFD) communication, ultra-wideband (UWB) communication, Ant+ communication, WIFI communication, radio frequency identification (RFID) communication, 3G communication, 4G communication, 5G communication, and the like. However, this is merely exemplary and embodiments are not limited thereto.

In addition, the action interface 130 may store processing results of the pulse wave sensor 110 and the processor 120, and various types of reference information required for predicting health problems in a storage. For example, the action interface 130 may manage a measurement history of pulse wave signals and an estimation history of optical path length changes by using the storage, and in response to a user's request, the action interface 130 may provide the history information from the storage. In this case, various types of reference information may include a correlation between the optical path length change and the variation in red blood cell size, a reference value for predicting whether there is a health problem, reference interval information for each level, a user's characteristic information, and the like.

Further, the storage may be embedded in a device including the healthcare apparatus 100, and/or a separate external device. The storage may include at least one storage medium of a flash memory type memory, a hard disk type memory, a multimedia card micro type memory, a card type memory (e.g., an SD memory, an XD memory, etc.), a random access memory (RAM), a static random access memory (SRAM), a read only memory (ROM), an electrically erasable programmable read only memory (EEPROM), a programmable read only memory (PROM), a magnetic memory, a magnetic disk, and an optical disk, and the like, but is not limited thereto.

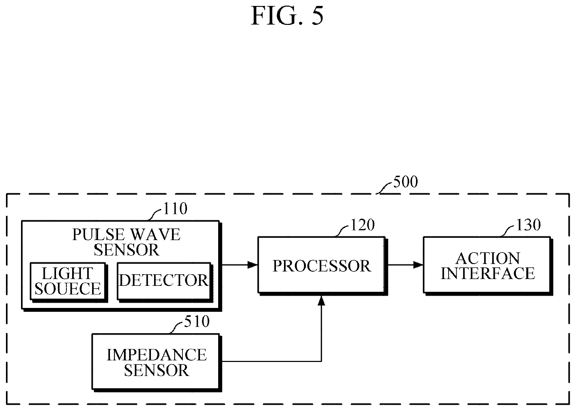

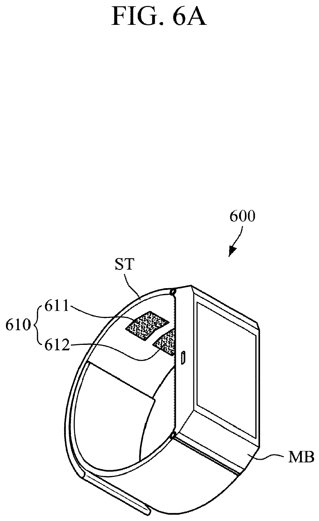

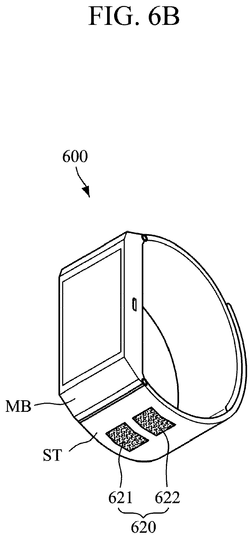

FIG. 5 is a block diagram illustrating a healthcare apparatus according to an example embodiment. FIGS. 6A and 6B are diagrams illustrating a wearable device including the healthcare apparatus of FIG. 5.

Referring to FIG. 5, the healthcare apparatus 500 according to an example embodiment includes the pulse wave sensor 110, the processor 120, the action interface 130, and an impedance sensor 510. The pulse wave sensor 110, the processor 120, and the action interface 130 are described in detail above, such that the following description will be given based on non-overlapping parts.

While the pulse wave sensor 110 measures a pulse wave signal from an examination portion of a user, the impedance sensor 510 may measure a user's bio-impedance. In this case, the impedance sensor 510 may measure bio-impedance by using a two-electrode method or a four-electrode method.

For example, FIGS. 6A and 6B illustrate a wearable device 600 including the healthcare apparatus 500. As illustrated in FIGS. 6A and 6B, the wearable device 600 may be a smart watch-type wearable device including a main body MB and a strap ST or a smart band-type wearable device, but is not limited thereto.

In the wearable device 600, the impedance sensor 510 may include a first electrode part 610 and a second electrode part 620 for obtaining an impedance signal by applying an alternating current and by measuring a voltage between both ends of the electrodes. The first electrode part 610 may be disposed inside of the strap ST and the second electrode part 620 may be disposed outside of the strap ST. However, the arrangement is not limited thereto, and the first electrode part 610 may be disposed on a rear surface of the main body MB which comes into contact with a user's wrist when the main body MB is worn on the wrist, and the second electrode part 620 may be disposed on a front surface of the main body MB However, embodiments are not limited thereto, and any one of the first electrode part 610 and the second electrode part 620 may be disposed at the main body MB, and the other one may be disposed at the strap ST. In this case, the electrode part disposed on a rear surface of the main body MB may be disposed around the pulse wave sensor 110.

As illustrated in FIGS. 6A and 6B, in order to measure impedance using the four-electrode method, the first electrode part 610 may include an input electrode 611 to which an alternating current is applied and an output electrode 612 for measuring a voltage between both ends of the electrodes. The second electrode part 620 may include an input electrode 621 to which an alternating current is applied, and an output electrode 622 for measuring a voltage between both ends of the electrodes. In this case, an arrangement direction of the input electrodes 611 and 612 and the output electrodes 612 and 622 may be perpendicular to a longitudinal direction of the strap ST. However, the input electrodes 611 and 612 and the output electrodes 612 and 622 are not limited thereto, and may be arranged in various shapes such as a circular shape, an oval shape, and the like according to an arrangement position of the main body MB or the strap ST.

When a user wears the main body MB to measure a pulse wave signal, the first electrode part 610 of the impedance sensor 510 may come into contact with the user's wrist. In this case, the user may contact the second electrode part 620 with another body part, e.g., a finger, a lateral side, the palm, and the wrist of the other hand, on which the main body MB is not worn. However, embodiments are not limited thereto, and the user may contact the second electrode part 620 with another body part, such as the chest, the leg, the neck, and the like, by moving the hand on which the main body MB is worn.

The impedance sensor 510 may measure a change in a user's bio-impedance by applying an alternating current within a predetermined frequency range. For example, the frequency range may be pre-defined to provide a difference in electrical characteristics of a user's body which result from balance between intracellular and extracellular fluid compartments. For example, the frequency range may be from 1 kHz to 1 MHz, but is not limited thereto.

The processor 120 may estimate a body fluid variation by using the user's bio-impedance measured by the impedance sensor 510. The processor 120 may estimate the body fluid variation according to the change in bio-impedance measured from the user based on a pre-defined correlation between the bio-impedance change and the body fluid variation.

Upon estimating the body fluid variation, the processor 120 may determine whether the user has a health problem based on the estimated body fluid variation. For example, in response to the estimated body fluid variation exceeding a predetermined reference value, the processor 120 may predict that the user is at risk of hyperosmolar coma. In another example, by using predetermined reference interval information for each level, the processor 120 may predict a risk level of hyperosmolar coma of a reference interval corresponding to the user's body fluid variation.

In yet another example, the processor 120 may linearly/non-linearly combine the body fluid variation with the optical path length change obtained based on the pulse wave signal, and may determine if there is a risk of hyperosmolar coma by comparing the combination result with a reference value or the reference interval information. In this case, a weighted value may be assigned to each of the body fluid variation and the optical path length change.

As described above, the action interface 130 may perform an appropriate action in response to determination as to whether a user is at risk of having a health problem.

In addition, the healthcare apparatus 500 may further include a sensor for obtaining additional information, such as a degree of perspiration, body temperature, heartbeat, blood pressure, and the like, from a user. The processor 120 may obtain additional information, such as blood pressure, heartbeat, skin elasticity, skin aging, and the like, based on the pulse wave signal measured by the pulse wave sensor 110. The processor 120 may obtain one or more features by analyzing a waveform of the pulse wave signal, and may obtain additional information, such as blood pressure and the like, by linearly/non-linearly combining the obtained one or more features. However, the method of obtaining the additional information is not limited thereto, and the additional information may be obtained based on the pulse wave signal by using various other methods.

The processor 120 may determine whether there is a health problem by further using the obtained additional information in addition to the optical path length change and the body fluid variation. For example, even when it is determined that there is a risk of hyperosmolar coma based on the optical path length change and/or the body fluid variation, if additional information indicates a normal health condition or is not related to symptoms of hyperosmolar coma, the processor 120 may determine that there is no risk of hyperosmolar coma, or may reduce a degree of risk of hyperosmolar coma. In another example, the processor 120 may combine all the optical path length change, the body fluid variation, and the additional information, and may determine whether there is a risk of hyperosmolar coma by comparing the combination result with a reference value, but the determination is not limited thereto.

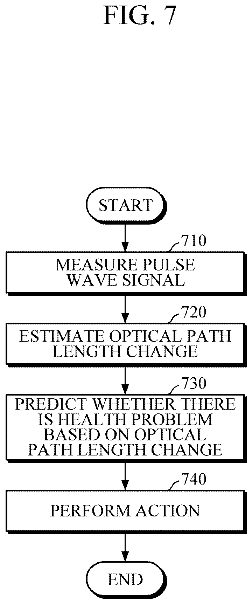

FIG. 7 is a flowchart illustrating a healthcare method according to an example embodiment. The healthcare method of FIG. 7 may be an example of a healthcare method performed by the healthcare apparatus 100 of FIG. 1.

In response to a user's request for predicting a health problem, or at predetermined intervals for continuous measurement, the healthcare apparatus 100 may measure a pulse wave signal from an examination portion of a user by using a pulse wave sensor in 710. In this case, the pulse wave sensor may emit light onto the examination portion, and may detect light scattered or reflected from the examination portion. The pulse wave sensor may obtain pulse wave signals of various wavelengths.

Then, the healthcare apparatus 100 may estimate an optical path length change by using the pulse wave signal in 720. The healthcare apparatus 100 may extract an AC component and/or a DC component by preprocessing the pulse wave signal, and may estimate the optical path length change by using the AC component and/or the DC component.

Subsequently, the healthcare apparatus 100 may predict whether there is a health problem based on the estimated optical path length change in 730. For example, based on a correlation between the optical path length change and the variation in red blood cell size, the healthcare apparatus 100 may monitor the variation in red blood cell size according to the optical path length change. In this case, if it is predicted for a diabetic patient that the size of red blood cells changes due to hyperosmotic pressure, the healthcare apparatus 100 may predict that there is a risk of hyperosmolar coma. A reference value may be pre-defined for the optical path length change and a health problem, for example, a risk of hyperosmolar coma, and if the estimated optical path length change exceeds the reference value, the healthcare apparatus 100 may predict that there is a risk of hyperosmolar coma.

Next, the healthcare apparatus 100 may perform an appropriate action in response to the predicted health problem in 740. For example, if there is a risk of hyperosmolar coma, the healthcare apparatus 100 may provide a warning for a user by using a display, a speaker, a haptic device, and the like. Further, the healthcare apparatus 100 may provide an action to be made urgently by a user in response to the health problem.

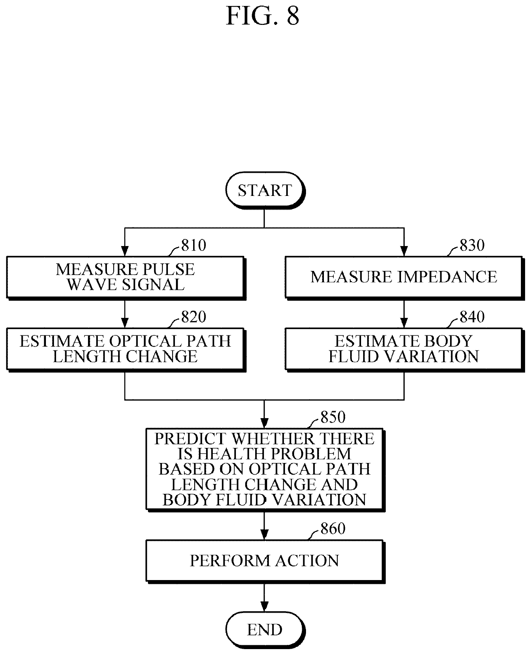

FIG. 8 is a flowchart illustrating a healthcare method according to an example embodiment. The healthcare method of FIG. 8 may be an example of a healthcare method performed by the healthcare apparatus 500 of FIG. 5.

The healthcare apparatus 500 may measure a pulse wave signal from an object portion of a user by using a pulse wave sensor in 810. In this case, the pulse wave sensor may emit light onto the examination portion, and may detect light scattered or reflected from the examination portion.

Then, the healthcare apparatus 500 may estimate an optical path length change by using the pulse wave signal in 820. The healthcare apparatus 500 may extract an AC component and/or a DC component by preprocessing the pulse wave signal, and may estimate the optical path length change by using the AC component and/or the DC component.

Further, the healthcare apparatus 500 may measure the user's bio-impedance by using an impedance sensor in 830.

Subsequently, the healthcare apparatus 500 may estimate a body fluid variation based on a correlation between a change in the measured bio-impedance and the body fluid variation in 840.

Next, the healthcare apparatus 500 may predict whether there is a health problem in 850 based on the optical path length change estimated in 820 and the body fluid variation estimated in 840. For example, the healthcare apparatus 500 may combine the optical path length change with the body fluid variation, and in response to the combination result exceeding a pre-defined reference value, the healthcare apparatus 500 may predict that there is a risk of hyperosmolar coma.

Then, the healthcare apparatus 500 may perform an appropriate action in response to the predicted health problem in 860. For example, if there is a risk of hyperosmolar coma, the healthcare apparatus 100 may provide a warning for a user by using a display, a speaker, a haptic device, and the like. Further, the healthcare apparatus 100 may provide an action to be made urgently by a user in response to the health problem.

Example embodiments can be realized as a computer-readable code written on a computer-readable recording medium. The computer-readable recording medium may be any type of recording device in which data is stored in a computer-readable manner.

Examples of the computer-readable recording medium include a ROM, a RAM, a CD-ROM, a magnetic tape, a floppy disc, an optical data storage, and a carrier wave (e.g., data transmission through the Internet). The computer-readable recording medium can be distributed over a plurality of computer systems connected to a network so that a computer-readable code is written thereto and executed therefrom in a decentralized manner. Functional programs, codes, and code segments needed for realizing the example embodiments can be readily deduced by programmers in the technical field to which the present disclosure pertains.

While one or more example embodiments have been described with reference to the figures, it will be understood by those of ordinary skill in the art that various changes in form and details may be made therein without departing from the scope as defined by the following claims.

**Claims**

1. A healthcare method comprising:

   measuring a pulse wave signal by emitting light onto an a user and detecting light reflected or scattered from the user;
   obtaining an optical path length change of blood based on the pulse wave signal; and
   predicting whether the user has a health problem based on the obtained optical path length change.

2. The method of claim 1, wherein the obtaining of the optical path length change comprises extracting an alternating current component of the pulse wave signal, and obtaining the optical path length change based on a maximum value of the extracted alternating current and a minimum value of the extracted alternating current component.

3. The method of claim 1 or 2, wherein the predicting whether the user has the health problem comprises predicting hyperosmolar coma by monitoring a change in red blood cell size of the user based on the obtained optical path length change.

4. The method of claim 3, wherein the predicting whether the user has the health problem comprises predicting that the user is at risk of hyperosmolar coma based on the obtained optical path length change exceeding a predetermined reference value.

5. The method of claim 4, wherein the predetermined reference value comprises reference interval information for each of a plurality of levels corresponding to a degree of risk of hyperosmolar coma, wherein the predicting whether the user has a health problem comprises predicting the degree of risk of hyperosmolar coma based on the obtained optical path length change based on the reference interval information for each of the plurality of levels.

6. The method of one of claims 1 to 5, further comprising performing an action corresponding to the health problem of the user based on the prediction that the user has the health problem.

7. The method of claim 6, wherein the performing of the action comprises outputting an alarm by at least one of a display, a speaker, and a haptic device based on the prediction that the user has the health problem, or
   wherein the performing of the action comprises outputting at least one of information on the predicted health problem of the user and an action corresponding to a degree of risk of the health problem based on the prediction that the user has the health problem, or
   wherein the performing of the action comprises transmitting information on the health problem to an electronic device by a communication interface based on the prediction that the user has the health problem.

8. The method of claim 7, wherein:

   the information on the predicted health problem comprises at least one of the optical path length change, impedance information, information on whether a blood glucose concentration is abnormal, information on whether there is a risk of hyperosmolar coma, and a degree of risk of hyperosmolar coma; and
   the action comprises at least one of medication intake, fluid intake, rest, exercise, and hospital information.

9. A healthcare apparatus, comprising:

   a pulse wave sensor comprising a light source configured to emit light onto a user and a detector configured to detect light reflected or scattered from the user, the pulse wave sensor being configured to measure a pulse wave signal based on the detected light;
   an impedance sensor configured to measure an impedance of the user; and
   a processor configured to:

      obtain an optical path length change of blood based on the pulse wave signal;
      obtain a body fluid variation based on the measured impedance; and
      predict whether the user has a health problem based on the optical path length change and the body fluid variation.

10. The apparatus of claim 9, wherein the processor is further configured to predict a risk of hyperosmolar coma by monitoring a variation in red blood cell size of the user based on the optical path length change and the body fluid variation.

11. The apparatus of claim 10, wherein the processor is further configured to combine the optical path length change and the body fluid variation, and wherein the processor is configured to predict that there is a risk of hyperosmolar coma based on the combination of the optical path length change and the body fluid variation exceeding a predetermined reference value.

12. The apparatus of claim 11, wherein the processor is further configured to assign a weighted value to each of the optical path length change and the body fluid variation based on at least one characteristic of the user, and to combine the optical path length change and the body fluid variation, to each of which the weighted value is assigned.

13. The apparatus of one of claims 9 to 12, further comprising an action interface configured to perform an action corresponding to the health problem of the user based on the prediction that the user has the health problem.

14. A computer-readable recording medium, having instructions that when performed by a processor of a healthcare apparatus a pulse wave sensor comprising a light source configured to emit light toward a user and a detector configured to detect light reflected or scattered from the user, the pulse wave sensor being configured to measure a pulse wave signal based on the detected light, cause the processor to:

    obtain an optical path length change of blood of the user based on the pulse wave signal; and predict a risk of hyperosmolar coma of the user by monitoring a variation in red blood cell size of the user based on the obtained optical path length change exceeding a predetermined reference value.

15. The computer-readable recording medium of claim 14, wherein the predetermined reference value corresponds to at least one characteristic of the user, the at least one characteristic including being diabetic and non-diabetic.

# FIG. 1

```
┌─ ─ ─ ─ ─ ─ ─ ─ ─ ─ ─ ─ ─ ─ ─ ─ ─ ─ ─ ─ ─ ─ ─ ─ ─ ─ ─ ─100─ ┐
│  ┌─────110──────────┐     ┌──120──┐      ┌──130──┐         │
│  │  PULSE WAVE       │     │       │      │       │         │
│  │    SENSOR         │     │PROCESSOR│ →  │ ACTION │         │
│  │ ┌─────┐ ┌───────┐ │  →  │       │      │INTERFACE│        │
│  │ │LIGHT│ │DETECTOR│ │    └───────┘      └───────┘         │
│  │ │SOUECE│ └───────┘ │                                     │
│  │ └─────┘           │                                      │
│  └──────────────────┘                                       │
└─ ─ ─ ─ ─ ─ ─ ─ ─ ─ ─ ─ ─ ─ ─ ─ ─ ─ ─ ─ ─ ─ ─ ─ ─ ─ ─ ─ ─ ─┘
```

# FIG. 2

# FIG. 3A

FIG. 3B

CONSTANT VALUE
K - LIGHT INTENSITY

$I_{Peak}$
(Systole)

$I_{ac}$ | ac

$I_{Onset}$
(Diastole) | dc

TIME

FIG. 4A

# FIG. 4B

400a

10:00 AM

PLEASE DRINK
WATER

410

# FIG. 4C

# FIG. 5

# FIG. 6A

# FIG. 6B

600

MB

ST

621    622

620

# FIG. 7

```
            ( START )
                |
                v          ,710
  +----------------------------+
  |     MEASURE PULSE          |
  |      WAVE SIGNAL           |
  +----------------------------+
                |
                v          ,720
  +----------------------------+
  |  ESTIMATE OPTICAL PATH     |
  |      LENGTH CHANGE         |
  +----------------------------+
                |
                v          ,730
  +----------------------------+
  | PREDICT WHETHER THERE      |
  |   IS HEALTH PROBLEM        |
  |    BASED ON OPTICAL        |
  |  PATH LENGTH CHANGE        |
  +----------------------------+
                |
                v          ,740
  +----------------------------+
  |     PERFORM ACTION         |
  +----------------------------+
                |
                v
             ( END )
```

FIG. 8

START

MEASURE PULSE
WAVE SIGNAL — 810

ESTIMATE OPTICAL PATH
LENGTH CHANGE — 820

MEASURE IMPEDANCE — 830

ESTIMATE BODY
FLUID VARIATION — 840

PREDICT WHETHER THERE
IS HEALTH PROBLEM
BASED ON OPTICAL PATH
LENGTH CHANGE AND
BODY FLUID VARIATION — 850

PERFORM ACTION — 860

END

Europäisches
Patentamt

European
Patent Office

Office européen
des brevets

# EUROPEAN SEARCH REPORT

**Application Number**

EP 20 16 5194

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2016/080911 A1 (UNIV NANYANG TECH [SG]) 26 May 2016 (2016-05-26) * page 7 - page 10 * * page 14 - page 15 * * page 21 - page 25 * * page 36 - page 43; claims; figures * | 1-15 | INV. A61B5/145 A61B5/1455 A61B5/053 A61B5/00 |
| X<br><br>A | US 9 820 690 B1 (SCHWARTZ JERROD JOSEPH [US] ET AL) 21 November 2017 (2017-11-21) * column 8, line 18 - column 9, line 57 * * column 12, line 14 - column 14, line 9 * * column 17, line 7 - column 21, line 3 * * column 27, line 50 - column 28, line 15; claims; figures * | 1-8,14, 15<br><br>9-13 | |
| X<br><br>A | US 2018/116604 A1 (NEWBERRY ROBERT STEVEN [US]) 3 May 2018 (2018-05-03) * paragraphs [0095] - [0128], [0158], [0167], [0178] - [0185], [0202], [0255] - [0273]; claims; figures * | 1,2,6-8<br><br>3-5,9-15 | |
| X<br><br>A | US 2019/069245 A1 (MILLER DEVIN W [US] ET AL) 28 February 2019 (2019-02-28) * paragraphs [0002], [0039] - [0040], [0076] - [0079], [0090], [0094], [0101], [0104] - [0106], [0125], [0137], [0147] - [0164] * * paragraphs [0172], [0176], [0191]; claims; figures * | 1,2,6-9, 13<br><br>3-5, 10-12, 14,15 | TECHNICAL FIELDS SEARCHED (IPC)<br><br>A61B |

-/--

The present search report has been drawn up for all claims

| Place of search | Date of completion of the search | Examiner |
|---|---|---|
| The Hague | 29 July 2020 | Crisan, Carmen-Clara |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

## EUROPEAN SEARCH REPORT

Application Number

EP 20 16 5194

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | OHAD COHEN ET AL: "Glucose Correlation with Light Scattering Patterns-A Novel Method for Non-Invasive Glucose Measurements", DIABETES TECHNOLOGY & THERAPEUTICS, vol. 5, no. 1, 1 February 2003 (2003-02-01), pages 11-17, XP055041859, ISSN: 1520-9156, DOI: 10.1089/152091503763816418 | 1-8,14, 15 | |
| A | * the whole document * | 9-13 | |

----

TECHNICAL FIELDS
SEARCHED    (IPC)

The present search report has been drawn up for all claims

| Place of search | Date of completion of the search | Examiner |
|---|---|---|
| The Hague | 29 July 2020 | Crisan, Carmen-Clara |

CATEGORY OF CITED DOCUMENTS

X : particularly relevant if taken alone
Y : particularly relevant if combined with another
 document of the same category
A : technological background
O : non-written disclosure
P : intermediate document

T : theory or principle underlying the invention
E : earlier patent document, but published on, or
 after the filing date
D : document cited in the application
L : document cited for other reasons

& : member of the same patent family, corresponding
 document

EPO FORM 1503 03.82 (P04C01)

**ANNEX TO THE EUROPEAN SEARCH REPORT
ON EUROPEAN PATENT APPLICATION NO.**

EP 20 16 5194

This annex lists the patent family members relating to the patent documents cited in the above-mentioned European search report.
The members are as contained in the European Patent Office EDP file on
The European Patent Office is in no way liable for these particulars which are merely given for the purpose of information.

29-07-2020

| Patent document cited in search report | | Publication date | Patent family member(s) | | Publication date |
|---|---|---|---|---|---|
| WO 2016080911 | A1 | 26-05-2016 | AU 2015350582 A1 | | 06-07-2017 |
| | | | EP 3220824 A1 | | 27-09-2017 |
| | | | JP 2017538474 A | | 28-12-2017 |
| | | | SG 11201704055Y A | | 29-06-2017 |
| | | | WO 2016080911 A1 | | 26-05-2016 |
| US 9820690 | B1 | 21-11-2017 | NONE | | |
| US 2018116604 | A1 | 03-05-2018 | US 2018116604 A1 | | 03-05-2018 |
| | | | US 2019167206 A1 | | 06-06-2019 |
| US 2019069245 | A1 | 28-02-2019 | US 10154460 B1 | | 11-12-2018 |
| | | | US 10307101 B1 | | 04-06-2019 |
| | | | US 10485475 B1 | | 26-11-2019 |
| | | | US 2019069245 A1 | | 28-02-2019 |
| | | | US 2019246977 A1 | | 15-08-2019 |

EPO FORM P0459

For more details about this annex : see Official Journal of the European Patent Office, No. 12/82